# EUROPÄISCHE PATENTSCHRIFT

(11) **EP 0 700 889 B1**
(45) Veröffentlichungstag und Bekanntmachung des Hinweises auf die Patenterteilung: **14.01.1998**
(21) Anmeldenummer: 95112365.2
(22) Anmeldetag: 05.08.1995
(51) Int. Cl.: C07C 43/315, C07C 41/56, C07C 41/50

(54) **Verfahren zur Herstellung von 1,1,3-Trialkoxypropan**
Process for the preparation of 1,1,3-trialkoxypropanes
Procédé pour la préparation de trialkoxy-1,1,3-propanes

(30) Priorität: 08.09.1994 DE 4431994
(43) Veröffentlichungstag der Anmeldung: 13.03.1996
(73) Patentinhaber: Degussa Aktiengesellschaft, 60311 Frankfurt (DE)
(72) Erfinder: Höpp, Mathias, Dr., D-63599 Biebergemünd-Kassel (DE); Arntz, Dietrich, Dr., D-61440 Oberursel (DE); Ohlinger, Hans-Peter, Dr., D-63477 Maintal (DE); Hofen, Willi, D-63517 Rodenbach (DE)

(56) Entgegenhaltungen:
- DE-C- 898 895
- DE-C- 941 974
- DE-C- 957 569
- FR-A- 1 447 138
- US-A- 2 288 211

## Beschreibung

Die Erfindung betrifft ein Verfahren zur Herstellung von 1,1,3-Trialkoxypropan durch säurekatalysierte Umsetzung von Acrolein mit einem niederen Alkohol. Das Verfahren eignet sich insbesondere zur kontinuierlichen Herstellung von 1,1,3-Trialkoxypropan mit hoher Selektivität.

Es ist bekannt, 1,1,3-Trialkoxypropan durch Umsetzung von Acrolein mit einem niederen Alkohol in Gegenwart von Salzsäure oder Schwefelsäure als Katalysator, teilweiser oder vollständiger Neutralisation des Reaktionsgemischs und destillativer Aufarbeitung desselben herzustellen (DE-PS 898 895; R.H. Hall und E.S. Stern in J.Chem. Soc., 3388 bis 3393 (1954)). Bei der säurekatalysierten Umsetzung von Acrolein (I) mit einem niederen Alkohol wird nicht nur das gewünschte 1,1,3-Trialkoxypropan (IV) gebildet, sondern es entstehen auch 3-Alkoxypropionaldehyd (II) und Acroleindialkylacetal (III). Wie aus dem nachfolgenden Reaktionsschema hervorgeht, bestehen zwischen den genannten Stoffen (I) bis (IV) in Gegenwart des sauren Katalysators Gleichgewichte:

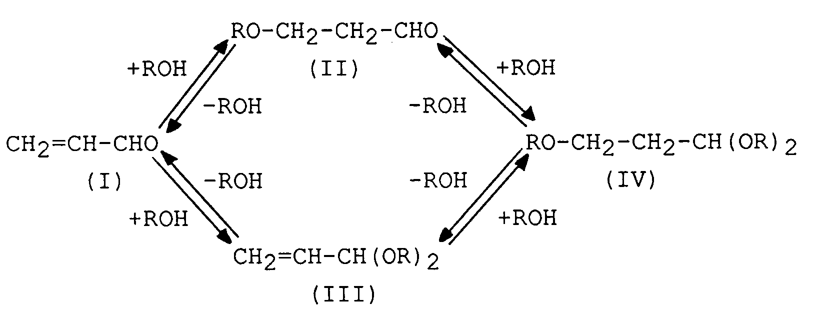

Aufgrund der komplexen Gleichgewichtslagen zwischen den Verbindungen (I) bis (IV) sowie der Tatsache, daß aus Acrolein und dem bei der Acetalbildung entstehenden Wasser weitere Acrolein-Folgeprodukte entstehen können, war es bisher schwierig, das gewünschte 1,1,3-Trialkoxypropan mit hoher Selektivität und hoher Gesamtausbeute zu erhalten. Um eine Rückspaltung von (IV) während der destillativen Aufarbeitung des Reaktionsgemischs möglichst zu vermeiden, ist zumindest eine Teilneutralisation des Reaktionsgemischs vor der Destillation erforderlich. Nachteilig an dem vorbekannten Verfahren ist neben der vielfach ungenügenden Ausbeute, daß es aufgrund der im Reaktionsgemisch enthaltenen Salze aus der Neutralisation des Katalysators zu Verkrustungen in den Destillationseinrichtungen kommt - eine derartige Verkrustung ist in einer großtechnischen Anlage nicht tolerabel.

Gemäß DE-PS 898 895 kann das Gleichgewicht der Umsetzung zugunsten des gewünschten Trialkoxypropans dadurch verschoben werden, daß die Umsetzung in Gegenwart wasserbindender Stoffe durchgeführt wird; eine solche Maßnahme ist aber technisch sehr aufwendig. In der DE-PS 898 895 wird auch gelehrt, den in Beispiel 3 als Nebenprodukt entstehenden 3-Methoxypropionaldehyd, der bei der destillativen Aufarbeitung im Gemisch mit Wasser erhalten wird, unter Verwendung von Benzol azeotrop zu entwässern und einem nächsten Ansatz zuzusetzen; nachteilig ist hierbei, daß zur Entwässerung ein weiteres organisches Lösungsmittel erforderlich ist.

Aufgabe der vorliegenden Erfindung ist demgemäß, ein verbessertes Verfahren zur Herstellung von 1,1,3-Trialkoxypropan aufzuzeigen, das problemlos in technischem Maßstab realisiert und batchweise oder kontinuierlich durchgeführt werden kann. Das erfindungsgemäße Verfahren sollte ferner zu einer höheren Selektivität an 1,1,3-Trialkoxypropan führen. Schließlich sollte sich der als Nebenprodukt gebildete 3-Alkoxypropionaldehyd ohne das Erfordernis der Mitverwendung eines weiteren organischen Lösungsmittels einem Folgeansatz zuführen lassen, um so zu einer hohen Ausbeute an 1,1,3-Trialkoxypropan zu gelangen.

Gefunden wurde ein Verfahren zur Herstellung von 1,1,3-Trialkoxypropan durch Umsetzung von Acrolein mit einem C₁- bis C₆-Alkohol im Molverhältnis von 1 zu größer 3 bei 10 bis 100 °C in Gegenwart eines sauren Katalysators, Teilneutralisation des Reaktionsgemischs und eine Destillation umfassende Auftrennung des teilneutralisierten Reaktionsgemischs, das dadurch gekennzeichnet ist, daß man (a) die Umsetzung in Gegenwart eines im Reaktionsgemisch unlöslichen festen sauren Katalysators durchführt, (b) den pH-Wert des vom Katalysator befreiten Reaktionsgemischs durch Zugabe eines Amins oder Kontaktieren mit einem basischen Ionenaustauscher auf 4,5 bis 7 erhöht, gemessen in einer auf das zehnfache mit Wasser verdünnten Probe, und (c) das teilneutralisierte Reaktionsgemisch destillativ in eine oder mehrere im wesentlichen alkoxypropionaldehydfreie Leichtsiederfraktionen und eine 3-Alkoxypropionaldehyd und 1,1,3-Trialkoxypropan enthaltende wasserfreie Hochsiederfraktion oder, sofern die Siedepunkte von Wasser, 3-Alkoxypropionaldehyd und des gemeinsamen Azeotrops eng beieinander liegen, in eine Leichtsiederfraktion, eine im wesentlichen die gesamten Mengen 3-Alkoxypropionaldehyd und Wasser enthaltende Mittelsiederfraktion und eine 1,1,3-Trialkoxypropan enthaltende Hochsiederfraktion auftrennt, 1,1,3-Trialkoxypropan aus der jeweiligen Hochsiederfraktion abdestilliert und den aus der Hochsiederfraktion abdestillierten oder aus der Mittelsiederfraktion durch Pervaporation zumindest teilweise entwässerten 3-Alkoxypropionaldehyd einem Folgeansatz zuführt.

Das erfindungsgemäß herzustellende 1,1,3-Trialkoxypropan enthält drei gleiche Alkoxygruppen mit 1 bis 6 C-Atomen. Bei der Herstellung des 1,1,3-Trialkoxypropans werden demgemäß Alkohole mit 1 bis 6 C-Atomen, vorzugsweise 1 bis 3 C-Atomen, eingesetzt; Bei den Alkoholen kann es sich um primäre oder sekundäre Alkohole handeln, nicht jedoch um teriäre Alkohole. Lineare Alkohole mit 1 bis 3 C-Atomen, also Methanol, Ethanol und n-Propanol, werden bevorzugt eingesetzt, weil die hiermit erhältlichen 1,1,3-Trialkoxypropane geeignete Rohstoffe zur Herstellung der als Lösungsmittel verwendbaren entsprechenden 1,3-Dialkoxypropane sind. Besonders bevorzugt wird 1,1,3-Triethoxypropan.

Zur Herstellung von 1,1,3-Trialkoxypropan werden Acrolein und Alkohol in einem Molverhältnis von 1 zu größer 3 eingesetzt, weil ein Alkoholüberschuß die Gleichgewichtslage in die gewünschte Richtung verschiebt; der obere Grenzwert für das Molverhältnis ist an sich wenig kritisch und wird, um das Verfahren nicht unwirtschaftlich zu gestalten, im allgemeinen kaum über 1 zu 20 liegen. Ein Molverhältnis von Acrolein zu Alkohol im Bereich zwischen 1 zu 3,5 bis 1 zu 10 und insbesondere zwischen 1 zu 4 bis 1 zu 8 wird bevorzugt. Zweckmäßigerweise sollten die einzusetzenden Rohstoffe möglichst wasserfrei sein oder nur einen geringen Wassergehalt aufweisen, weil es sonst zu einer ungünstigeren Gleichgewichtseinstellung und zu vermehrter Nebenproduktbildung kommt.

Die Umsetzung erfolgt in einem Temperaturbereich zwischen 10 und 100 °C, vorzugsweise zwischen 30 und 70 °C. Bei einer Umsetzungstemperatur im Bereich der oberen Grenze wird das Gleichgewicht mehr zur Seite der Einsatzstoffe verschoben und zudem werden vermehrt Nebenprodukte gebildet; bei sehr niedriger Temperatur ist die Reaktionsgeschwindigkeit gegebenenfalls zu gering, um eine ausreichend hohe Raum-Zeit-Ausbeute zu erzielen. Üblicherweise erfolgt die Umsetzung bei Normaldruck; soweit erwünscht oder bei höherer Temperatur erforderlich, kann auch ein erhöhter Druck eingestellt werden.

Als Katalysator werden feste, im Reaktionsgemisch unlösliche saure Katalysatoren verwendet. Einsetzbar sind insbesondere anorganische und organische Ionenaustauscher. Verwendbar sind aber auch anorganische Trägermaterialien mit hoher Oberfläche, welche eine starke Mineralsäure ausreichend fest adsorbiert enthalten - die Säure darf sich während der Umsetzung praktisch nicht von der Katalysatoroberfläche ablösen.

Unter den organischen Ionenaustauschern sind Austauscherharze auf der Basis von Styrol/Divinylbenzol-Copolymeren mit Sulfonat- oder Phosphonatgruppen, wobei solche mit stärker sauren Sulfonatgruppen bevorzugt werden, besonders geeignet. Zweckmäßig ist es, makroporöse Ionenaustauscher zu verwenden. Auch marktübliche perfluorierte Sulfonsäureharze sind einsetzbar.

Unter den sauren anorganischen Ionenaustauschern werden bevorzugt solche auf der Basis polymerer Organosiloxane mit Sulfonatgruppen gemäß DE-PS 35 18 881 und DE-PS 32 26 093, ferner saure Zeolithe, deren SiO₂/Al₂O₃-Modul größer 2 ist, beispielsweise Zeolithe vom Typ Y, Mordenit und ZSM 5, sowie Schichtsilikate mit sauren Zwischenschichten, etwa mit Mineralsäuren vorbehandelte Montmorillonite, verstanden.

Das erfindungsgemäß gebildete Reaktionsgemisch weist nach Einstellung des Gleichgewichts reaktionsbedingt einen pH-Wert im Bereich von im allgemeinen 3,0 bis 6,0 auf, meist von 3,0 bis 4,5, jeweils gemessen in 10-facher Verdünnung mit Wasser. Um während der Aufarbeitung des Reaktionsgemischs eine Rückspaltung des gebildeten 1,1,3-Trialkoxypropans zu vermeiden, muß der pH-Wert zunächst auf einen Wert zwischen 4,5 und 7,0, vorzugweise zwischen 5,5 und 7,0 und insbesondere zwischen 6,0 und 7,0, jeweils gemessen in 10-facher Verdünnung mit Wasser, erhöht werden. Da ein pH-Wert im Reaktionsgemisch von exakt 7,0 in der Technik nur schwer zu realisieren ist, ein pH-Wert oberhalb 7 aber auf alle Fälle zu vermeiden ist, wird in der Praxis ein pH-Wert von knapp 7 bevorzugt. pH-Werte über 7 sind im Reaktionsgemisch zu vermeiden, da sonst spontane Polymerisation des darin enthaltenen Acroleins erfolgt. Sofern der pH-Wert des Reaktionsgemischs am Ende der Umsetzung bei oder über 4,5 liegt, kann gegebenenfalls auf eine pH-Erhöhung verzichtet werden. Zur pH-Erhöhung wird vorzugsweise ein Amin, insbesondere ein tertiäres Amin, eingesetzt. Bevorzugt werden tertiäre Amine, deren Siedepunkt oberhalb des Siedepunkts des gewünschten 1,1,3-Trialkoxypropans liegt, da diese hochsiedenden Amine die destillative Aufarbeitung des Reaktionsgemischs und die Rückführung einzelner Fraktionen in einen Folgeansatz nicht negativ beeinflussen. Besonders geeignete hochsiedende tertiäre Amine sind Trialkanolamine. Als Alternative zur pH-Erhöhung kann auch das Reaktionsgemisch mit einem basischen Ionenaustauscher kontaktiert werden, etwa einem stark basischen organischen Ionenaustauscher.

Das destillativ aufzuarbeitende Reaktionsgemisch besteht im wesentlichen aus 1,1,3-Trialkoxypropan (IV), 3-Alkoxypropan (II), dem entsprechenden Alkanol und Wasser. Unter dem Begriff "im wesentlichen" wird verstanden, daß das Reaktionsgemisch zusätzliche Bestandteile enthält, wie nicht-umgesetztes Acrolein, Acroleindialkylacetal (III) und Nebenprodukte, die durch Michael-Addition eines Moleküls Wasser an zwei Moleküle Acrolein und anschließende Aldolkondensation sowie Acetalisierung gebildet werden. Die Zusammensetzung des Reaktionsgemischs richtet sich nach dem gewählten Molverhältnis Acrolein zu Alkanol, dem Wassergehalt der eingesetzten Edukte und der Reaktionstemperatur und entspricht vorzugsweise der jeweiligen Gleichgewichtszusammensetzung.

Das erfindungsgemäße Verfahren läßt sich diskontinuierlich, beispielweise in einem Rührreaktor, oder kontinuierlich, beispielsweise in einem Schlaufenreaktor, wobei der Katalysator als Festbett oder Fließbett angeordnet sein kann, durchführen. Prinzipiell sind alle Reaktorformen geeignet, welche einen ausreichenden Kontakt zwischen dem Reaktionsgemisch und dem festen Katalysator gewährleisten. Bei diskontinuierlicher Umsetzung wird vorzugsweise der Alkohol mit dem sauren Katalysator vorgelegt und Acrolein bei der eingestellten Reaktionstemperatur zugetropft. Nach einer Nachreaktionszeit wird abgekühlt und der Ionenaustauscher abfiltriert. Bei kontinuierlicher Verfahrensweise werden kontinuierlich Acrolein und Ethanol im gewünschten Molverhältnis zum Reaktionsgemisch eingespeist und die entsprechende Menge Reaktionsgemisch zur Aufarbeitung abgezogen. Zweckmäßigerweise werden auch aus der Aufarbeitung des Reaktionsgemischs zurückgewonnene Rohstoffe, wie Acrolein und Alkohol, und die Acrolein-Nebenprodukte (III) und (IV) zum Reaktionsgemisch eingespeist. Für die Kontaktzeit des Reaktionsgemischs, ausgedrückt durch den LHSV-Wert (Liquid Hourly Space Velocity) gilt, daß der Wert zwischen 0,5 und 30 und vorzugsweise zwischen 1 und 15 liegen sollte.

Gemäß einer bevorzugten Ausführungsform erfolgt die Umsetzung in einem Umlaufreaktor (Schlaufenreaktor), der einen Behälter mit dem Katalysatorbett, eine Umlaufleitung sowie Vorrichtungen zum Zuführen der Reaktanden und Abnehmen des Reaktionsgemischs enthält. Um das Gleichgewicht in die gewünschte Richtung zu verschieben, hat es sich als vorteilhaft erwiesen, in die Umlaufleitung eine Vorrichtung zur teilweisen Entwässerung des Reaktionsgemischs zu integrieren. Zur Entwässerung des Reaktionsgemischs eignet sich eine Pervaporationsvorrichtung; unter Verwendung einer hydrophilen Membran permeiert Wasser, und das teilweise entwässerte Reaktionsgemisch verbleibt auf der Retentatseite. Einrichtungen zur Pervaporation und Maßnahmen zur Durchführung derselben sind dem Fachmann bekannt - siehe Ullmann's Encyclopedia of Industrial Chemisty 5.ed. Vol. A 16 (1990), 208 und Vol. A 9 (1987), 638 und die dort zitierte Literatur.

Die destillative Aufarbeitung des zuvor mindestens teilweise neutralisierten Reaktionsgemischs erfolgt in Abhängigkeit der Siedepunkte des als Nebenprodukt entstehenden 3-Alkoxypropionaldehyds und dessen Azeotrops mit Wasser:

Sofern die Siedepunkte von Wasser, 3-Alkoxypropionaldehyd und des gemeinsamen Azeotrops eng beieinanderliegen, dies ist insbesondere der Fall bei 3-Methoxypropionaldehyd, wird das Reaktionsgemisch in eine Leichtsiederfraktion, welche nicht-umgesetztes Acrolein und den Alkoholüberschuß enthält, eine im wesentlichen die gesamte Menge 3-Alkoxypropionaldyd und die gesamte Menge Wasser enthaltende Mittelsiederfraktion und eine das gewünschte 1,1,3-Trialkoxypropan und hochsiedende Nebenprodukte enthaltende Hochsiederfraktion aufgetrennt. Da 1,1,3-Trialkoxypropan in Gegenwart von Wasser auch thermisch rückspaltbar ist, sollte die Abtrennung der Mittelsiederfraktion möglichst rasch und unter schonenden Bedingungen erfolgen. 1,1,3-Trialkoxypropan wird aus der Hochsiederfraktion abdestilliert. Zwecks Rückführung des 3-Alkoxypropionaldehyds in einen Folgeansatz wird die wäßrige Mittelsiederfraktion unter Verwendung einer Pervaporationsvorrichtung mit einer hydrophilen Membran zumindest teilweise entwässert.

Bei der Herstellung von 1,1,3-Trialkoxypropan, deren Alkoxygruppen zwei oder mehr als zwei Kohlenstoffatome aufweisen, hat es sich als besonders zweckmäßig erwiesen, das Reaktionsgemisch derart destillativ aufzutrennen, daß zunächst eine, gegebenenfalls auch mehrere, im wesentlichen alkoxypropionaldehydfreie Leichtsiederfraktionen mit den Hauptkomponenten Acrolein, Alkohol und Wasser abzutrennen und aus der verbleibenden wasserfreien Hochsiederfraktion 3-Alkoxypropionaldehyd und 1,1,3-Trialkoxypropan abzudestillieren. Bei der Herstellung von Triethoxypropan werden vorteilhafterweise sämtliche Leichtsieder in einer Fraktion abgenommen; die Leichtsiederfraktion enthält zudem das als Nebenprodukt im Gleichgewichtssystem enthaltene Acroleindiethylacetal.

Vor der erneuten Einspeisung der in dem wäßrigen Leichtsiedergemisch enthaltenen Wertstoffe Acrolein und Alkohol, muß ein Teil des Wassers ausgeschleust werden. Dies kann durch Destillation erfolgen, wobei aber im Falle eines ethanolischen Leichtsiedergemischs wegen der Azeotropbildung Ethanol/Wasser ein Restwassergehalt von etwa 4 % im Destillat verbleibt; während das Destillat rezykliert werden kann, muß der wäßrige Sumpf, der auch Acroleindiethylacetal enthält, entsorgt werden.

Es wurde nun gefunden, daß sich wäßrige Leichtsiedergemische in vorteilhafter Weise durch Pervaporation auf Restwassergehalt unter 1 % entwässern lassen. Hierbei wird das Leichtsiedergemisch bei einer Temperatur von 40 bis 105 °C und vorteilhafterweise bei 60 bis 100 °C über eine hydrophile Membran geleitet und das Permeat bei einem Druck von 0,1 bis 50 mbar, vorteilhafterweise 1 bis 30 mbar, gasförmig abgezogen. Für diese wie auch die bereits zuvor genannten Pervaporationsmaßnahmen sind alle Arten von hydrophilen Membranen einsetzbar, wie beispielsweise Polyvinylalkoholmembranen oder Symplexkompositmembranen. Ein wesentlicher Vorteil der Entwässerung wäßriger Leichtsiedergemische als auch wäßriger Mittelsiedergemische durch Pervaporation besteht darin, daß weder ein weiteres organisches Lösungsmittel zwecks azeotroper Entwässerung noch ein Trocknungsmittel erforderlich sind. Ein weiterer Vorteil besteht darin, daß das in einer wäßrigen Leichtsiederfraktion gegebenenfalls enthaltene Acroleindialkylacetal bei der pervaporativen Entwässerung nicht verloren geht, sondern mit der entwässerten Leichtsiederfraktion zusammen mit dem Alkohol und nichtumgesetzten Acrolein rezykliert werden kann. Hierdurch wird die Ausbeute erhöht und die Entsorgungskosten sinken.

Das erfindungsgemäße Verfahren läßt sich diskontinuierlich und kontinuierlich im technischen Maßstab durchführen, ohne das es in den Vorrichtungen zur Destillation zu Verkrustungen kommt. Es sind nur sehr geringe Mengen an Neutralisationsmittel erforderlich, da die festen sauren Katalysatoren im Reaktionsmilieu unlöslich sind. Bei der Verwendung der bevorzugten tertiären Amine als Neutralisationsmittel verbleiben die sich bildenden Ammoniumsalze in nicht-kristalliner Form im Destillationssumpf bei der Aufarbeitung im Reaktionsgemisch.

Überraschenderweise konnte die Selektivität an 1,1,3-Trialkoxypropan und insbesondere an 1,1,3-Triethoxypropan durch die Art und Weise der destillativen Auftrennung des Reaktionsgemischs nennenswert gesteigert werden. Schließlich ist als weiterer Vorteil die einfache Entwässerung wäßriger Fraktionen aus der Aufarbeitung des Reaktionsgemischs und Rückführung der zumindestens teilweise entwässerten Phasen in den nächsten Reaktionsansatz hervorzuheben. Durch Integration einer Pervaporationsstufe in einen Umlaufreaktor kann zusätzlich das Gleichgewicht verschoben werden, so daß das aus dem Kreislauf abgezogene Reaktionsgemisch einen höheren Gehalt an 1,1,3-Trialkoxypropan enthält und die Menge an rückzuführenden Komponenten reduziert werden kann; hierdurch sinken die Betriebs- und Anlagenkosten.

### Beispiel 1

1150 g absolutes Ethanol und 40 g stark saurer Ionenaustauscher (Amberlyst® 15 Fa. Rohm & Haas) werden vorgelegt und auf 50 °C aufgewärmt. 292 g Acrolein (96 %ig) werden innerhalb von 30 Minuten zudosiert, wobei die Innentemperatur im Bereich 50 bis 53 °C gehalten wird. Nach beendeter Zugabe wird 4 h bei 50 °C Innentemperatur nachgerührt. Anschließend wird noch 2 h bei 20 °C nachgerührt. Der Ionentauscher wird abfiltriert und das Reaktionsgemisch wird mit ethanolischer Triethanolaminlösung versetzt, bis eine Probe des Reaktionsgemischs nach 10-facher Verdünnung mit Wasser einen pH-Wert >6 und <7 aufweist.

Das Reaktionsgemisch wird durch Destillation aufgearbeitet, wobei mittels einer 70 cm-Kolonne, gefüllt mit Maschendrahtringen, bei 67 bis 79 °C Kopftemperatur und einem Druck von 1000 mbar zunächst ein Leichtsiedergemisch aus Ethanol Wasser, Acrolein und Acroleindiethylacetal abdestilliert wird. Anschließend werden aus der verbleibenden Hochsiederfraktion bei 65 bis 68 °C und 88 mbar zunächst 120 g 3-Ethoxypropionaldehyd (II) (23 %) und anschließend 92 bis 94 °C und 50 mbar 585 g 1,1,3-Triethoxypropan (IV) (67 %) abdestilliert.

### Beispiel 2

a) Die Durchführung der Reaktion erfolgt wie in Beispiel 1, jedoch wird Ethanol mit einem Wassergehalt von 4 % eingesetzt. Wegen der gegenüber Beispiel 1 verschlechterten Gleichgewichtslage und der erhöhten Nebenproduktbildung liefert die destillative Aufarbeitung, durchgeführt wie in Beispiel 1, 130 g 3-Ethoxypropionaldehyd (25 %) und 440 g 1,1,3-Triethoxypropan (50 %).
b) Nicht-erfindungsgemäße Aufarbeitung:
   Die Durchführung der Reaktion erfolgt wie im Beispiel 2a. Das Reaktionsgemisch wird über eine gut trennende Kolonne mit 15 theoretischen Böden destilliert. Zunächst wird bei 78 °C und 1000 mbar ein Leichtsiedergemisch mit einem Wassergehalt von 4,8 % abdestilliert, anschließend bei 43 °C und 100 mbar ein Gemisch aus Wasser und 3-Ethoxypropionaldehyd und zum Schluß bei 101 °C und 50 mbar 1,1,3-Triethoxypropan. (Das Azeotrop Ethoxypropionaldehyd/Wasser (42,5 % H₂O) siedet bei Normaldruck bei 93 °C). Man erhält 102 g 3-Ethoxypropionaldehyd (20 %), im Gemisch mit Wasser vorliegend, und 302 g 1,1,3-Triethoxypropan (35 %). Der Acrolein-Anteil im Leichtsiedergemisch beträgt 5,0 %, während er im Beispiel 2a nur bei 1,5 % lag. Dies und die verminderte Ausbeute belegen eine Zersetzung während der Destillation.

### Beispiel 3

Die Durchführung der Reaktion und die destillative Aufarbeitung erfolgten wie in Beispiel 1. Das abgetrennte Leichtsiedergemisch hat eine Masse von 686 g und setzt sich wie folgt zusammen: 0,6 % Acrolein, 10,2 % Wasser, 87,6 % Ethanol und 1,6 % Acroleindiethylacetal (III).

Dieses Gemisch wird mit einem Volumenstrom von 100 l/h über 100 cm² eine Membran (Pervap® 1000 der Fa. Deutsche Carbone) gepumpt. Der Betrieb erfolgt batchweise bei einer Temperatur von 80 °C und einem Permeatdruck von 10 mbar. Das Permeat wird mit flüssigem Stickstoff auskondensiert. Nach 10 h Betriebsdauer erhält man 84 g Permeat mit einem Wassergehalt von 73 % (26 % EtOH) und 584 g Retentat mit einem Wassergehalt von 0,5 %, 0,7 % Acrolein, 97 % Ethanol und 1,7 % (III).

Bezogen auf die Einsatzstoffe erhält man einen Umsatz für Acrolein von 98,5 %, für Ethanol von 50,7 %. Die Selektivität bezüglich (IV) liegt für Acrolein bei 67,5 %, für Ethanol bei 78,7 %. Die Summenselektivität bezüglich (IV), (II) und (III) liegt für Acrolein bei 93,0 %, für Ethanol bei 89 %. Berücksichtigt man den Wiedereinsatz von (II) und (III) bei Folgeansätzen, erhält man bei einem kontinuierlichen Prozeß eine Selektivität bezüglich (IV) für Acrolein von 90 % und für Ethanol von 88 %.

### Beispiel 4

In einer kontinuierlich betriebenen Versuchsanlage mit einem Festbettreaktor werden stündlich folgende Mengen umgesetzt und aufgearbeitet:

1235 g Acrolein (96 %ig) und 2865 g Ethanol werden in einen Vormischer gepumpt, zusammen mit 5455 g Retentat aus der Pervaporationsstufe (5380 g Ethanol, 25 g Wasser, 20 g ADEA (=III) und 30 g Acrolein) und 430 g EPA (=II) aus der 2. Destillationsstufe. Dieses Gemisch wird kontinuierlich bei 50 °C über ein Katalysatorfestbett mit 2000 g stark saurem Kationenaustauscher (Amberlyst® 15) gepumpt. In das abfließende Reaktionsgemisch werden zur Neutralisation kontinuierlich 40 g einer 50 %igen Lösung von Triethanolamin in Ethanol gepumpt.

Das neutralisierte Reaktionsgemisch wird in einer 1. Destillationskolonne bei Normaldruck von den Leichtsiedern befreit. Man erhält 5840 g Destillat (5400 g EtOH, 390 g Wasser, 20 g ADEA und 30 g Acrolein) und 4055 g Rückstand (430 g EPA, 3280 g TREP (=IV), 345 g Hochsieder).

Das Destillat wird auf 100 °C erwärmt in eine Pervaporationseinheit eingespeist und dort entwässert. Die Membranfläche beträgt 1 m² Pervap® 1516. Das Permeat wird mit Kühlsole bei -20 °C auskondensiert. Pro Stunde erhält man 485 g Permeat (365 g Wasser, 120 g EtOH) und 5355 g Retentat, welches wieder in den Vormischer der Reaktion eingespeist wird.

Der Rückstand der 1. Destillation wird einer 2. Destillation bei 100 mbar unterworfen. Hierbei werden 430 g EPA abdestilliert und ebenfalls in den Vormischer zurückgeführt. Der Rückstand der 2. Destillation wird bei 50 mbar nochmals destilliert, wobei 3280 g 1,1,3-Triethoxypropan erhalten werden.

Die Selektivität bezüglich TREP beträgt für das Acrolein 88 % und für das Ethanol 89,8 %.

### Beispiel 5

800 g Methanol und 30 g saurer Ionenaustauscher (Amberlyst® 15) werden vorgelegt und auf 50 °C aufgewärmt. 292 g Acrolein (96 %ig) werden innerhalb von 30 Minuten zudosiert, wobei die Innentemperatur im Bereich 50 bis 53 °C gehalten wird. Nach beendeter Zugabe wird 4 h bei 50 °C Innentemperatur nachgerührt. Anschließend wird abgekühlt, der Ionentauscher abfiltriert und das Reaktionsgemisch mit methanolischer Triethanolaminlösung versetzt, bis das Reaktionsgemisch nach zehnfacher Verdünnung mit Wasser einen pH-Wert von 6,5 aufweist.

Das Reaktionsgemisch wird destillativ aufgearbeitet. In der ersten Fraktion werden nicht-umgesetztes Acrolein (8 g) und Methanol (380 g) abdestilliert, die direkt wieder für den nächsten Einsatz verwendet werden können. In der zweiten Fraktion wird ein Gemisch aus Wasser (76 g) und Methoxypropionaldehyd (85 g) sowie etwas Methanol (15 g) und Trimethoxypropan (17 g) abdestilliert. Die dritte Fraktion enthält 465 g reines Trimethoxypropan.

Die 193 g der zweiten Fraktion werden mit einem Volumenstrom von 100 l/h über 100 cm² eine Membran (Pervap® 1000 der Fa. Deutsche Carbone) gepumpt. Der Betrieb erfolgt batchweise bei einer Temperatur von 80 °C und einem Permeatdruck von 10 mbar. Das Permeat wird mit flüssigem Stickstoff auskondensiert. Nach 10 h Beriebsdauer erhält man 92 g Permeat mit einem Gehalt von 82 % Wasser und 15 % Methanol und 97 g Retentat mit einem Gehalt von 0,5 % Wasser, 83 % Methoxypropionaldehyd und 16 % Trimethoxypropan. Das Retentat kann wieder für die nächste Reaktion verwendet werden.

Bezogen auf die Einsatzstoffe erhält man einen Umsatz für Acrolein von 97 %, für Methanol von 52 %. Berücksichtigt man den Wiedereinsatz des Retentats und der Fraktion 1, so erhält man bei einem kontinuierlichen Prozeß eine Selektivität bezüglich Trimethoxypropan für Acrolein von 91 % und für Methanol von 88 %.

## Patentansprüche

1. Verfahren zur Herstellung von 1,1,3-Trialkoxypropan durch Umsetzung von Acrolein mit einem C₁- bis C₆-Alkohol im Molverhältnis von 1 zu größer 3 bei 10 bis 100 °C in Gegenwart eines sauren Katalysators, Teilneutralisation des Reaktionsgemischs und eine Destillation umfassende Auftrennung des teilneutralisierten Reaktionsgemischs,
dadurch gekennzeichnet,
daß man (a) die Umsetzung in Gegenwart eines im Reaktionsgemisch unlöslichen festen sauren Katalysators durchführt, (b) den pH-Wert des vom Katalysator befreiten Reaktionsgemischs durch Zugabe eines Amins oder Kontaktieren mit einem basischen Ionenaustauscher auf 4,5 bis 7 erhöht, gemessen in einer auf das zehnfache mit Wasser verdünnten Probe, und (c) das teilneutralisierte Reaktionsgemisch destillativ in eine oder mehrere im wesentlichen alkoxypropionaldehydfreie Leichtsiederfraktionen und eine 3-Alkoxypropionaldehyd und 1,1,3-Trialkoxypropan enthaltende wasserfreie Hochsiederfraktion oder, sofern die Siedepunkte von Wasser, 3-Alkoxypropionaldehyd und des gemeinsamen Azeotrops eng beieinander liegen, in eine Leichtsiederfraktion, eine im wesentlichen die gesamten Mengen 3-Alkoxypropionaldehyd und Wasser enthaltende Mittelsiederfraktion und eine 1,1,3-Trialkoxypropan enthaltende Hochsiederfraktion auftrennt, 1,1,3-Trialkoxypropan aus der jeweiligen Hochsiederfraktion abdestilliert und den aus der Hochsiederfraktion abdestillierten oder aus der Mittelsiederfraktion durch Pervaporation zumindest teilweise entwässerten 3-Alkoxypropionaldehyd einem Folgeansatz zuführt.

2. Verfahren nach Anspruch 1,
dadurch gekennzeichnet,
daß man Acrolein und Alkohol im Molverhältnis von 1 zu 4 bis 1 zu 8 einsetzt.

3. Verfahren nach Anspruch 1 oder 2,
dadurch gekennzeichnet,
daß man Acrolein mit Methanol, Ethanol oder n-Propanol, vorzugsweise Ethanol, umsetzt.

4. Verfahren nach einem oder mehreren der Ansprüche 1 bis 3,
dadurch gekennzeichnet,
daß man zur Teilneutralisation ein tertiäres Amin mit einem Siedepunkt oberhalb des Siedepunkts des 1,1,3-Trialkoxypropans verwendet.

5. Verfahren nach einem oder mehreren der Ansprüche 1 bis 4,
dadurch gekennzeichnet,
daß man als Katalysator einen stark sauren organischen oder anorganischen Ionenaustauscher verwendet.

6. Verfahren nach einem oder mehreren der Ansprüche 1 bis 5,
dadurch gekennzeichnet,
daß man die Umsetzung bei 30 bis 70 °C durchführt.

7. Verfahren nach einem oder mehreren der Ansprüche 1 bis 6,
dadurch gekennzeichnet,
daß man zwecks Umsetzung ein Acrolein und Alkohol enthaltendes Gemisch über einen sauren Festbettkatalysator leitet und das erhaltene Reaktionsgemisch vor oder nach der Teilneutralisation vor der destillativen Aufarbeitung mittels Pervaporation unter Verwendung einer hydrophilen Membran zumindest teilweise entwässert.

8. Verfahren nach Anspruch 7,
dadurch gekennzeichnet,
daß man die Umsetzung in einem Umlaufreaktor, umfassend einen Behälter mit dem Katalysatorbett, eine Pervaporationsvorrichtung, eine Umlaufleitung und Vorrichtungen zum Zuführen der Reaktanden und Abnehmen des teilweise entwässerten Reaktionsgemischs, durchführt.

9. Verfahren nach einem oder mehreren der Ansprüche 1 bis 8,
dadurch gekennzeichnet,
daß man eine Wasser enthaltende Leichtsiederfraktion mittels Pervaporation unter Verwendung einer hydrophilen Membran entwässert und das Alkohol und/oder Acrolein enthaltende Retentat der Pervaporation in einen Folgeansatz rezykliert.

10. Verfahren nach einem der Ansprüche 7 bis 9,
dadurch gekennzeichnet,
daß man in der Pervaporationsstufe eine Membran aus Polyvinylalkohol verwendet.

## Claims

1. Process for the preparation of 1,1,3-trialkoxypropane by the reaction of acrolein with a C₁ to C₆ alcohol in the molar ratio of from 1 to greater than 3 at a temperature of from 10°C to 100°C in the presence of an acid catalyst, partial neutralisation of the reaction mixture and a separation comprising distillation of the partly neutralised reaction mixture,
characterised in that
(a) the reaction is carried out in the presence of a solid acid catalyst which is insoluble in the reaction mixture, (b) the pH value of the reaction mixture liberated from the catalyst is increased to 4.5 to 7, measured on a sample diluted with ten times the quantity of water, by addition of an amine or contact with a basic ion exchanger, and (c) the partly neutralised reaction mixture is separated by distillation into one or more low-boiling fractions substantially free of alkoxypropionaldehyde and an anhydrous high-boiling fraction containing 3-alkoxypropionaldehyde and 1,1,3-trialkoxypropane or, if the boiling points of water, of 3-alkoxypropionaldehyde and of the combined azeotropic mixture are close to one another, into a low-boiling fraction, a medium-boiling fraction containing substantially the entire quantity of 3-alkoxypropionaldehyde and water and a high-boiling fraction containing 1,1,3-trialkoxypropane, the 1,1,3-trialkoxypropane is distilled off from the high-boiling fraction in each case and the 3-alkoxypropionaldehyde distilled off from the high-boiling fraction or at least partly dehydrated by pervaporation from the medium-boiling fraction is fed to a subsequent batch.

2. Process according to claim 1,
characterised in that
acrolein and alcohol are used in the molar ratio of from 1 to 4 up to 1 to 8.

3. Process according to claim 1 or 2,
characterised in that
acrolein is reacted with methanol, ethanol or n-propanol, preferably ethanol.

4. Process according to one or more of claims 1 to 3,
characterised in that
a tertiary amine having a boiling point above the boiling point of the 1,1,3-trialkoxypropane is used for the partial neutralisation.

5. Process according to one or more of claims 1 to 4,
characterised in that
the catalyst used is a strongly acidic organic or inorganic ion exchanger.

6. Process according to one or more of claims 1 to 5,
characterised in that
the reaction is carried out at from 30 to 70°C.

7. Process according to one or more of claims 1 to 6,
characterised in that
for the purpose of the reaction, a mixture containing acrolein and alcohol is passed over an acidic fixed bed catalyst and the reaction mixture obtained, before or after the partial neutralisation prior to the working up by distillation, is at least partly dehydrated by means of pervaporation using a hydrophilic membrane.

8. Process according to claim 7,
characterised in that
the reaction is carried out in a circulating reactor comprising a container housing the catalyst bed, a pervaporation device, a circulation system and devices for introducing the reactants and withdrawing the partly dehydrated reaction mixture.

9. Process according to one or more of claims 1 to 8,
characterised in that
a low-boiling fraction containing water is dehydrated by means of pervaporation using a hydrophilic membrane and the retentate of the pervaporation, which contains alcohol and/or acrolein, is recycled to a subsequent batch.

10. Process according to any one of claims 7 to 9,
characterised in that
a membrane made of polyvinyl alcohol is used in the pervaporation step.

## Revendications

1. Procédé de production de 1,1,3-trialkoxypropane par mise en réaction de l'acroléine avec un alcool en C₁ à C₆, dans un rapport molaire de 1 à plus de 3, de 10 à 100°C en présence d'un catalyseur acide, neutralisation partielle du mélange réactionnel et séparation englobant une distillation du mélange réactionnel neutralisé partiellement,
caractérisé en ce que
a) on effectue la réaction en présence d'un catalyseur acide, solide, insoluble dans le mélange réactionnel,
b) on augmente la valeur du pH du mélange réactionnel dépourvu de catalyseur par addition d'une amine ou par mise en contact avec un échangeur d'ions basique de 4, 5 à 7, mesuré dans un échantillon dilué dix fois avec de l'eau, et
c) on sépare le mélange réactionnel neutralisé en partie, par distillation en une ou plusieurs fractions à bas point d'ébullition essentiellement dépourvues d'alkoxypropionaldéhyde et en une fraction à haut point d'ébullition anhydre, contenant du 3-alkoxypropionaldéhyde et du 1,1,3-trialkoxypropane ou pour autant que les points d'ébullition de l'eau, du 3-alkoxypropionaldéhyde et de l'azéotrope commun se situent étroitement l'un près de l'autre, en une fraction à bas point d'ébullition, une fraction à point d'ébullition médian contenant les quantités totales pour l'essentiel de 3-alkoxypropionaldéhyde et d'eau et une fraction à point d'ébullition élevé contenant le 1,1,3-trialkoxypropane, on sépare par distillation le 1,1,3-trialkoxypropane de la fraction à point d'ébullition élevé respective, ou on amène le 3-alkoxypropionaldéhyde au moins partiellement déshydraté, provenant de la fraction à point d'ébullition médian, par pervaporation à un lot suivant.

2. Procédé selon la revendication 1,
caractérisé en ce qu'
on met en oeuvre l'acroléine et l'alcool dans un rapport molaire allant de 1 à 4 à 1 à 8.

3. Procédé selon la revendication 1 ou la revendication 2,
caractérisé en ce qu'
on utilise l'acroléine avec du méthanol, de l'éthanol ou du n-propanol, de préférence avec l'éthanol.

4. Procédé selon une ou plusieurs des revendications 1 à 3,
caractérisé en ce qu'
on utilise pour la neutralisation partielle une amine tertiaire ayant un point d'ébullition au-dessus du point d'ébullition du 1,1,3-trialkoxypropane.

5. Procédé selon une ou plusieurs des revendications 1 à 4,
caractérisé en ce qu'
on utilise comme catalyseur un échangeur d'ions minéral ou organique, fortement acide .

6. Procédé selon une ou plusieurs des revendications 1 à 5,
caractérisé en ce qu'
on effectue la réaction de 30 à 70°C.

7. Procédé selon une ou plusieurs des revendications 1 à 6,
caractérisé en ce qu'
on conduit en vue de la réaction un mélange contenant de l'acroléine et un alcool, sur un catalyseur en lit fixe acide, et en ce qu'on déshydrate au moins partiellement le mélange réactionnel obtenu avant ou après la neutralisation partielle, avant le traitement par distillation à l'aide d'une pervaporation en utilisant une membrane hydrophile.

8. Procédé selon la revendication 7,
caractérisé en ce qu'
on effectue la réaction dans un réacteur à circulation, comprenant un récipient avec le lit de catalyseur, un dispositif de pervaporation, une conduite de dérivation et des dispositifs d'amenée des produits de réaction et d'évacuation du mélange réactionnel partiellement déshydraté.

9. Procédé selon une ou plusieurs des revendications 1 à 8,
caractérisé en ce qu'
on déshydrate une fraction à bas point d'ébullition contenant de l'eau à l'aide de pervaporation en utilisant une membrane hydrophile et on recycle dans un lot suivant le rétentat contenant l'alcool et l'acroléine de la pervaporation.

10. Procédé selon une des revendications 7 à 9,
caractérisé en ce qu'
on utilise dans l'étape de pervaporation une membrane en alcool polyvinylique.
